(19) Europäisches Patentamt
European Patent Office
Office européen des brevets

(11) Veröffentlichungsnummer: **0 101 582**
**B1**

# (12) EUROPÄISCHE PATENTSCHRIFT

(45) Veröffentlichungstag der Patentschrift: **16.07.86**

(51) Int. Cl.⁴: **C 07 D 241/40**, C 07 D 241/44, A 23 K 1/16, A 61 K 31/495

(21) Anmeldenummer: **83107636.9**

(22) Anmeldetag: **03.08.83**

(54) **Lichtstabilisierte Chinoxalin-di-N-oxide.**

(30) Priorität: **14.08.82 DE 3230273**

(43) Veröffentlichungstag der Anmeldung: **29.02.84 Patentblatt 84/9**

(45) Bekanntmachung des Hinweises auf die Patenterteilung: **16.07.86 Patentblatt 86/29**

(84) Benannte Vertragsstaaten: **AT BE CH DE FR GB IT LI NL SE**

(56) Entgegenhaltungen:
**DE - A - 1 954 226**
**DE - A - 2 147 545**
**DE - A - 2 615 646**
**US - A - 3 908 008**
**US - A - 4 171 355**

(73) Patentinhaber: **BAYER AG, Konzernverwaltung RP Patentabteilung, D-5090 Leverkusen 1 Bayerwerk (DE)**

(72) Erfinder: **Voege, Herbert, Dr., Martin-Buber-Strasse 41, D-5090 Leverkusen 3 (DE)**
Erfinder: **Sieveking, Hans Ulrich, Dr., An den Ruthen 4, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 101 582 B1

ACTORUM AG

**Beschreibung**

Die Erfindung betrifft lichtstabilisierte wässrige Lösungen von Chinoxalin-di-N-oxiden, enthaltend chemische Substanzen, die Licht im Wellenbereich von 200 bis 450 nm absorbieren.

Antibiotisch wirksame Chinoxalin-di-N-oxide werden vornehmlich als Wachstumsförderer oder als Arzneimittel in der Tiermedizin verwendet. Bei der Aufzucht von Geflügel ist die Applikation von Arzneimitteln über das Trinkwasser der Vögel üblich, desgleichen aber auch bei Schweinen u.a. Dazu ist eine Lösung des Wirkstoffes im Wasser vorteilhaft, weil eine homogene Verteilung gewährleistet ist und sich zum Beispiel keine Wirkstoffteilchen in der Tränke absetzen und verloren gehen oder Düsen verstopfen können.

Lösungen der Chinoxalin-di-N-oxide in Wasser sind aber nicht sehr lichtstabil. Unter Lichteinfluss wird der Wirkstoff — je nach Lichtintensität und Konzentration des Wirkstoffes — in weniger als 1 Stunde über die Hälfte zerstört.

Es besteht daher ein grosses Interesse daran, Lösungen dieser Wirkstoffklasse gegenüber dem Lichteinfluss zu stabilisieren.

Die Stabilisierung wässriger Wirkstoff-Formulierungen durch Lichtschutzmittel z.B. UV-Absorber ist bekannt. So ist z.B. in der US-PS 4 171 355 eine Pyrethroid-Formulierung zur Bekämpfung von Zecken beschrieben, die durch Zusatz von Benzophenon-Derivaten stabilisiert wird.

In der DE-OS 2 615 646 wird ebenfalls die zusätzliche Stabilisierung von mikroverkapseltem Pyrethroid durch Zusatz von Benzophenon-Derivaten beschrieben.

A.F. Asker et al. beschreiben die Licht-Stabilisierung von parenteralen Reserpin-Lösungen durch p-Aminobenzoesäure [Pharmazie 26, 89-92, (1971)].

Die Erfindung betrifft wässrige Chinoxalin di-N--oxid-Lösungen, die chemische Substanzen aus den Klassen der Chinoline, Flavone, Isoxanthenone oder Azofarbstoffe mit Absorptionsbereichen von 200 bis 450 nm enthalten und deren Menge das 0,1 bis 10fache des eingesetzten Chinoxalin-di-N-oxids der Formel I beträgt.

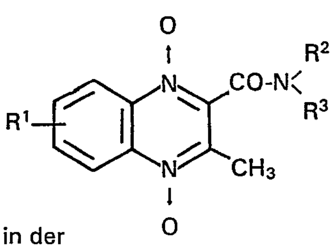

(I)

in der

$R^1$ für ein Wasserstoff- oder Chloratom oder eine niedere Alkyl- oder niedere Alkoxygruppe steht,

$R^2$ für einen gegebenenfalls durch eine Hydroxy-, niedere Alkoxy-, niedere Alkylamino- oder niedere Dialkylaminogruppe substituierten geradkettigen oder verzweigten Alkylrest mit 1 bis 6 Kohlenstoffatomen oder die Gruppe

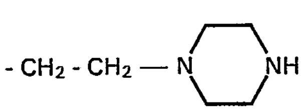

steht und

$R^3$ für ein Wasserstoffatom oder einen gegebenenfalls durch eine Hydroxy-, niedere Alkoxy-, niedere Alkylamino- oder niedere Dialkylaminogruppe substituierten geradkettigen oder verzweigten Alkylrest mit 1 bis 6 Kohlenstoffatomen steht und worin $R^2$ und $R^3$ gemeinsam mit dem Amidstickstoffatom, an das sie gebunden sind, einen Pyrrolidino-, Piperidino-, Morpholino- oder Piperazinorest bilden können; wobei der letztere in 4-Stellung durch einen niederen Alkylrest substituiert sein kann, der seinerseits durch eine Hydroxygruppe substituiert sein kann,

und wobei man unter niederen Alkyl- oder Alkoxygruppen stets solche mit 1 bis 4 Kohlenstoffatomen versteht.

Bevorzugt sind wässrige Chinoxalin-di-N-oxid-Lösungen, enthaltend chemische Substanzen, die Licht im Wellenbereich von 300 bis 400 nm absorbieren und, gemessen an einer 100 ppm Chinoxalin-di--N-oxid und 100 ppm absorbierende Substanz enthaltenden wässrigen Lösung bei 1stündiger Bestrahlung mit UV-Licht der Wellenlänge 366 nm (Desagalampe für Dünnschichtplatten Abstand 15 cm) den Abbau des Chinoxalin-di-N-oxids um mindestens 50% verringern.

Besonders bevorzugt enthalten die erfindungsgemässen Präparationen das auch unter dem Namen Olaquindox bekannte Chinoxalin-di-N-oxid der Formel

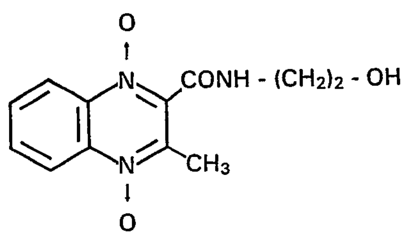

das in der Tierernährung eine bedeutende Rolle spielt.

Da die Wirkstoffe in einem Bereich von 25 ppm bis 500 ppm Verwendung finden, resultiert bei einem Einsatz der Farbstoffe in diesem Mengenbereich ein Lichtschutz.

Die schützenden Substanzen werden den Wirkstoffen oder den bestehenden Formulierungen z.B. wasserlöslichen Pulver oder Suspensionen einfach zugemischt. Natürlich können sie auch getrennt dem Wasser zugefügt werden.

Die folgenden Beispiele dienen zum Verdeutlichen der Erfindung.

*Material und Methoden*

1. Bestrahlungsbedingungen

Wässrige Lösungen mit 100 ppm Olaquindox wurden unter standardisierten Bedingungen bestrahlt: Hierzu wurden 100 ml Lösung in offenen Kristallisationsschalen (∅: 7,5 cm, Schichtdicke: 2,2 cm) der Lichtquelle (Abstand 15 cm) ausgesetzt.

2. Strahlungsquelle und -dauer

a) UV-Licht (langwellig. 366 nm) einer Desaga-Lampe (für Dünnschichtplatten), Abstand zur Probe: 10 cm, Strahlungsdauer: 1 h.

3. Konzentration der Additive

100 ppm
10 ppm

4. Analysenbedingungen

Der Wirkstoffgehalt wurde mittels DC-UV bestimmt, im allgemeinen gelang die Abtrennung von den Zusätzen mit Chloroform-Methanol (10+1) als Fliessmittel. Im Falle von Fluoreszein wurde Ethylacetat-Aceton-Ethanol-Diethylamin (50 + 40 + 10 + 5) verwendet. Bei sehr starkem Wirkstoffabbau wurde der Wirkstoffgehalt visuell abgeschätzt (Fleckvergleich auf DC-Platte), bei Konzentrationen 15% des Sollwertes wurde das Eluat vermessen.

Es wurden die in der folgenden Tabelle gezeigten Ergebnisse erhalten.

Tabelle

| Lichtschutz-Additiv | Konz. (ppm) | UV-Licht (366) 1 h Olaquindox-Gehalt (% v. Anfang 100 ppm) |
|---|---|---|
| Olaquindox ohne Lichtschutz | — | 15 |
| Quiuroniumsulfat (generic Name) | 100 | 39 |
| Tartrazin XX (Colour Index 2956, 19140) | 100 | 29 |
| 3,5,7,3',4'-Pentahydroxyflavon-3-rutinosid (=Rutin=Vitamin P) wasserlöslich | 100 | 30 |
| Fluorescein-Natriumsalz | 100 | 34 |
| Alizarinsulfonsaures Natrium | 100 | 33 |

**Patentansprüche**

1. Wässrige Chinoxalin-di-N-oxid-Lösungen, dadurch gekennzeichnet, dass sie gelöste chemische Substanzen aus den Klassen der Chinoline, Flavone, Isoxanthenone oder Azofarbstoffe enthalten, die Licht im Wellenbereich von 200 bis 450 nm absorbieren und deren Menge das 0,1 bis 10fache des eingesetzten Chinoxalin-di-N-oxids der allgemeinen Formel I beträgt

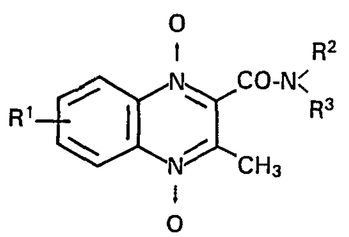

(I)

worin

$R^1$ für ein Wasserstoff- oder Chloratom oder eine niedere Alkyl- oder niedere Alkoxygruppe steht

$R^2$ für einen gegebenenfalls durch eine Hydroxy-, niedere Alkoxy-, niedere Alkylamino- oder niedere Dialkylaminogruppe substituierten geradkettigen oder verzweigten Alkylrest mit 1 bis 6 Kohlenstoffatomen oder die Gruppe

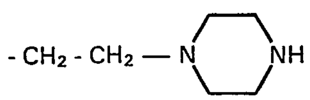

steht und

$R^3$ für ein Wasserstoffatom oder einen gegebenenfalls durch eine Hydroxy-, niedere Alkoxy-, niedere Alkylamino- oder niedere Dialkylaminogruppe substituierten geradkettigen oder verzweigten Alkylrest mit 1 bis 6 Kohlenstoffatomen steht und worin $R^2$ und $R^3$ gemeinsam mit dem Amidstickstoffatom, an das sie gebunden sind, einen Pyrrolidino-, Piperidino-, Morpholino- oder Piperazinorest bilden können; wobei der letztere in 4-Stellung durch einen niederen Alkylrest substituiert sein kann, der seinerseits durch eine Hydroxygruppe substituiert sein kann, und

wobei man unter niederen Alkyl- oder Alkoxygruppen stets solche mit 1 bis 4 Kohlenstoffatomen versteht.

2. Wässrige Chinoxalin-di-N-oxid-Lösungen nach Anspruch 1, dadurch gekennzeichnet, dass sie die Verbindung der Formel

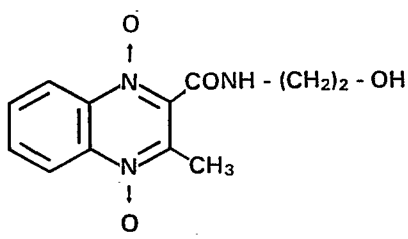

enthalten.

3. Verwendung von wässrigen Chinoxalin-di-N-oxid-Lösungen nach Anspruch 1 bei der Tieraufzucht.

**Claims**

1. Aqueous quinoxaline di-N-oxide solutions, characterised in that they contain dissolved chemical substances from the classes comprising the quinolines, flavones, isoxanthenones and azo dyes, which absorb light within the wave range of 200 to

450 nm and the quantity of which is 0.1 to 10 times that of the quinoxaline di-N-oxide used of the general formula I

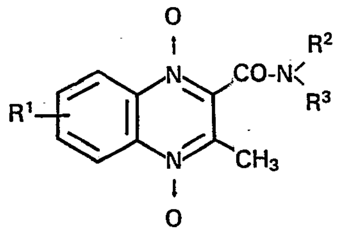

(I)

wherein

$R^1$ represents a hydrogen or chlorine atom or a lower alkyl or lower alkoxy group,

$R^2$ represents a straight-chain or branched alkyl radical which has 1 to 6 carbon atoms and is optionally substituted by a hydroxyl, lower alkylamino or lower dialkylamino group, or the group

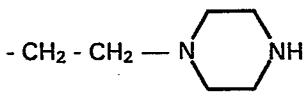

and

$R^3$ represents a hydrogen atom or a straight-chain or branched alkyl radical which has 1 to 6 carbon atoms and is optionally substituted by a hydroxyl, lower alkoxy, lower alkylamino or lower dialkylamino group, and wherein $R^2$ and $R^3$, together with the amide nitrogen atom to which they are bonded, can form a pyrrolidino, piperidino, morpholino or piperazino radical; it being possible for the latter to be substituted in the 4-position by a lower alkyl radical which in turn can be substituted by a hydroxyl group,

lower alkyl or alkoxy groups being understood in all cases to mean those with 1 to 4 carbon atoms.

2. Aqueous quinoxaline di-N-oxide solutions according to claim 1, characterised in that they contain the compound of the formula

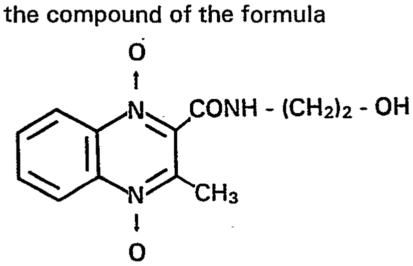

3. Use of aqueous quinoxaline di-N-oxide solutions according to claim 1 in rearing animals.

**Revendications**

1. Solutions aqueuses de di-N-oxydes de quinoxalines, caractérisées en ce qu'elles contiennent à l'état dissous des substances chimiques des classes des quinoléines, des flavones, des izoxanthénones ou des colorants azoïques, absorbant la lumière dans l'intervalle des longueurs d'onde de 200 à 450 nm, et dont la quantité représente de 0,1 à 10 fois la quantité du di-N-oxyde de quinoxaline mis en oeuvre, qui répond à la formule générale I

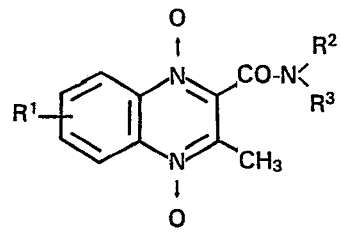

(I)

dans laquelle

$R^1$ représente un atome d'hydrogène ou de chlore, ou un groupe alkyle inférieur ou alcoxy inférieur,

$R^2$ représente un groupe alkyle droit ou ramifié en $C_1$-$C_6$ éventuellement substitué par un groupe hydroxy, alcoxy inférieur, alkylamino inférieur ou dialkylamino inférieur, ou le groupe

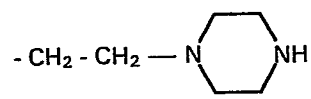

et

$R^3$ représente un atome d'hydrogène ou un groupe alkyle droit ou ramifié en $C_1$-$C_6$ éventuellement substitué par un groupe hydroxy, alcoxy inférieur, alkylamino inférieur ou dialkylamino inférieur, $R^1$ et $R^2$ pouvant former ensemble et avec l'atome d'azote d'amide auquel ils sont reliés un reste pyrrolidino, pipéridino, morpholino ou pipérazino; ce dernier pouvant être substitué en position 4 par un groupe alkyle inférieur lui-même éventuellement substitué par un groupe hydroxy, les groupes alkyle ou alkoxy qualifiés d'inférieurs étant toujours des groupes en $C_1$-$C_4$.

2. Solutions aqueuses de di-N-oxydes de quinoxalines selon la revendication 1, caractérisées en ce qu'elles contiennent le composé de formule

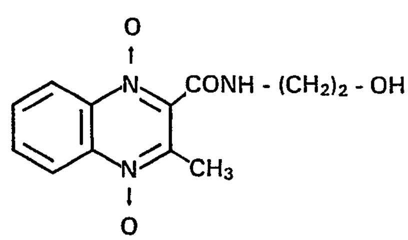

3. Utilisation des solutions aqueuses de di-N-oxydes de quinoxalines selon la revendication 1 dans l'élevage des animaux.